# EUROPÄISCHE PATENTSCHRIFT

(11) **EP 0 889 727 B1**
(45) Veröffentlichungstag und Bekanntmachung des Hinweises auf die Patenterteilung: **08.03.2006**
(21) Anmeldenummer: 97920535.8
(22) Anmeldetag: 11.03.1997
(51) Int. Cl.: A61K 31/565, A61P 5/30

(54) **SEQUENTIELLE ESTROGEN/PROGESTERONANTAGONIST-KOMBINATION FÜR DIE HORMONERSATZ-THERAPIE**
SEQUENTIAL OESTROGEN/PROGESTERONE ANTAGONIST COMBINATION FOR HORMONE REPLACEMENT THERAPY
COMBINAISON SEQUENTIELLE OESTROGENE/ANTAGONISTE DE LA PROGESTERONE S'UTILISANT EN THERAPIE HORMONALE DE SUBSTITUTION

(30) Priorität: 11.03.1996 DE 19610635
(43) Veröffentlichungstag der Anmeldung: 13.01.1999
(73) Patentinhaber: Schering Aktiengesellschaft, 13353 Berlin (DE)
(72) Erfinder: CHWALISZ, Kristof, Dr., D-13503 Berlin (DE)
(86) Internationale Anmeldenummer: PCT/DE1997/000580
(87) Internationale Veröffentlichungsnummer: WO 1997/033589

(56) Entgegenhaltungen:
- WO-A-94/18983

## Beschreibung

Die vorliegende Erfindung betrifft ein Arzneimittel enthaltend in Kombination einzelne Dosierungseinheiten eines Estrogens und einzelne Dosierungseinheiten eines kompetitiven Progesteronantagonisten zu dessen getrennter, sequentieller Verabreichung, eine Packung für die Hormonersatz-Therapie, enthaltend einzelne Dosierungseinheiten eines Estrogens und einzelne Dosierungseinheiten eines kompetitiven Progesteronantagonisten zu dessen getrennter, sequentieller Verabreichung sowie die Verwendung des vorstehend genannten Arzneimittels für die Hormonersatz-Therapie.

Mit Eintritt der Menopause (Klimakterium) bei Frauen treten aufgrund der veränderten Hormonproduktion sogenannte klimakterische Beschwerden auf. Durch die verminderte Estrogenproduktion steigt gleichzeitig das Osteoporose-Risiko (Verminderung des Knochengewebes bei erhaltener Knocbenstruktur durch gesteigerten Knochenabbau und/oder verminderten -anbau); ebenso wird bei postmenopausalen Frauen eine gegenüber prämenopausalen Frauen deutlich erhöhte Herzinfarktsrate sowie ein erhöhtes Auftreten anderer kardiovaskulärer Erkrankungen beobachtet, was ebenfalls auf die verminderte Estrogenproduktion zurückgeführt wird.

Die Hormonsubstitutions-Therapie (hormon replacement therapy = HRT) mit Estrogenen oder mit einer Estrogen/Gestagen-Kombination ist bisher die gängige Methode, um die mit der Menopause verbundenen Symptome zu therapieren (Ernster VL et al. (1988): Benefits and risks of menopausal estrogen and/or progestin hormone use; Prev. Med. 17:201-223).

Das Estrogen übt eine protektive Wirkung auf das kardiovaskuläre System, auf die Knochen (Verminderung des Osteoporose-Risikos) und auf das zentrale Nervensystem (Vermeidung sogenannter "hot-flashes") aus. Andererseits fuhrt die chronische Anwendung von Estrogenen in der Hormonersatz-Therapie zur Erhöhung des Risikos der Ausbildung eines Endometriumkarzinoms (Ernster VL et al. (1988): Benefits and risks of menopausal estrogen and/or progestin hormone use; Prev. Med. 17:201-223).

Durch die gleichzeitige Verwendung eines Gestagens zur Hormonsubstitutions-Therapie wird zwar der stimulierende Effekt des Estrogens auf das Endometrium unterbunden (Gibbson WE, 1986, Biochemical and histologic effects of sequential estrogen/progestin therapy on the endometrium of posunenopausal women; Am. J. Obstet. Gynecol: 154:46-61) andererseits können aber bei der kombinierten Therapie mit einem Estrogen und Gestagen die protektiven effekte der estrogenen Komponente hinsichtlich der Plasmalipide zumindest abgeschwächt werden (Lobo R. (1992): The role of progestins in hormone replacement therapy; Am. J. Obstet. Gynecol. 166:1997-2004).

Außerdem können unter einer Estrogen/Gestagenbehandlung aufgrund der im Vergleich zu einem oralen Kontrazeptivum verringerten Hormondosierung unerwünschte Zwischenblutungen auftreten (Hillard TC et al. (1992): Continuous combined conjugated equine estrogen-progestogen therapy: Effects of medroxyprogesterone acetate and norethindrone acetate on bleeding patterns and endometrial histologic diagnosis; Am. J. Obstet. Gynecol. 167: 1-7).

Schließlich zeigen neuere Befunde, daß manche Gestagene das Risiko der Ausbildung einer Brostkrebserkrankung erhöhen können (Staffa JA et al. (1992): Progestins and breast cancer: an epidemiologic review; 57: 473-491); King RJB (1991): A discussion of the roles of estrogen and progestin in human mammary carcinogenesis ; J. Ster. Biochem. Molec. Bio. 39: 8111-8118).

Zusammenfassend ergibt sich das Bild, daß die bekannte Estrogen-mono- sowie Estrogen/Gestagenkombinationstherapien keine befriedigenden Möglichkeiten zur Behandlung der mit der Menopause verbundenen Symptome darstellen.

Kürzlich ist auch die Verwendung "echter" Antiestrogene zur Herstellung von Arzneimitteln für die Hormonersatztherapie (HRT) vorgeschlagen worden (EP-A-0 178 862). Unter "echten" Antiestrogenen sind gemäß EP-A-0 178 862 beispielsweise Tamoxifen, Nafoxidin, MER-25 gemeint, also solche Antiestrogene, die rezeptorvermittelt wirken und die gleichzeitig noch eine estrogene (agonistische) Partialwirkung besitzen. Diese estrogene Partialwirkung tritt am Uterus und am Knochen auf.

Nachteilig bei einem derartigen, ein "echtes" Antiestrogen mit partialer estrogener Wirkung enthaltenden Arzneimittel ist, daß, bedingt durch die chronische estrogene Stimulation des Endometriums, wie bei der Anwendung von Estrogenen, ein erhöhtes Risiko der Entstehung eines Endomethumkarzinoms besteht (Fornander T et al. (1989): Adjuvant tarnoxifen in early breast cancer: occurrence of new primary cancers: Lancet 21: 117-119).

Andererseits zeigen sich durch die estrogene Partialwirkung von Tamoxifen positive Effekte auf die Knochen; Tamoxifen scheint bei Frauen den Abbau der Knochenmasse teilweise zu verhindern (Love RR et al. (1992): Effects of tamoxifen on bone mineral density in postmenopausal women with breast cancer; N. Engl. J. Med. 26:852-856).

Außerdem haben Untersuchungen mit Tamoxifen gezeigt, daß dessen antiestrogene Komponente für die Wachstumshemmung beim Einsatz in der Therapie des Mammakarzinoms bei postmenopausalen Frauen verantwortlich ist (Buckley MMT et al. (1989); Tamoxifen: A reappraisal of its pharmacodynamic and pharmacokinetic properties and therapeutic use; drugs 37: 451-490).

Weiterhin sind Antiestrogene vom Raloxifen-Typ zur Hemmung des Knochen-Abbaus sowie zur Behandlung des peri-menopausalen Syndroms bekannt geworden (US Patent 5,393, 763 bzw. 5,391,557). Antiestrogene dieses Typs zeigen eine deutlich reduzierte agonistische (estrogene) Wirkung am Endometrium, bewirken aber eine deutliche estrogene Wirkung am Knochen. Da aber auch diese Substanzen nicht vollständig dissoziert sind (d.h. sie haben immer noch eine estrogene Restwirkung am Endometrium), können auch sie zu einer Proliferation des Endometriums nach einer Langzeitbehandlung führen.

Demnach ist die erforderliche chronische Anwendung eines Antiestrogens mit agonistischer Partialwirkung in der Hormonsubstitutions-Therapie als bedenklich anzusehen, da eine Stimulation des Endometriums die Entstehung eines Endometriumkarzinoms begünstigen kann.

In der PCT-EP94/03408 wird vorgeschlagen, diese dauerhafte Stimulation des Endometriums durch die gleichzeitige Verwendung einer Verbindung mit progesteronantagonistischer sowie einer Verbindung mit antiestrogener bei gleichzeitiger partialer agonistischer Wirkung zur Herstellung eines Arzneimittels für die Hormonersatz-Therapie zu vermeiden. Bei einem solchen Arzneimittel inhibiert die Komponente mit progesteronantagonistischer Wirkung die durch die partiale estrogene Wirkung des Antiestrogens verursachten Veränderungen (Stimulation des Myo- und Endometriums) lediglich im Uterus, während jedoch die anderen, in der Hormonersatz-Therapie überaus erwünschten estrogenen Effekte, beispielsweise am Knochen und auf das kardiovaskuläre System, erhalten bleiben.

Die Verabreichung eines Estrogens, gegebenenfalls zusammen mit einem Gestagen, beide in sehr niedrigen Dosierungen, die alleine kein stabiles Blutungsverhalten gewährleisten, kombiniert mit einer periodischen, einmaligen Gabe eines Antiprogestins (Progesteronantagonisten) zur Kontrazeption und zur Hormonersatz-Therapie, ist in der WO-A 93/17686 beschrieben. Durch den Progesteronantagonisten kommt es zur Reduktion von Durchbruchblutungen.

Die gemeinsame, vorzugsweise gleichzeitige Verwendung eines kompetitiven Progesteronantagonisten mit einem Estrogen ohne Gestagen geht aus der WO-A 94/18983 hervor. Die Verwendung des Estrogens gemäß dieser Druckschrift geschieht vollkommen nach konventionellen Gesichtspunkten der Estrogenersatz-Therapie. Der Progesteronantagonist wird in einer Menge verwendet, die die durch das Estrogen induzierte endometriale Proliferation hemmt.

In jedem Fall kann eine chronische (z.B. tägliche) Behandlung mit einem Progesteronantagonisten aufgrund der täglichen Belastung des Organismus zu Nebenwirkungen, beispielsweise in der Leber, führen.

Aufgabe der vorliegenden Erfindung ist es, ein Arzneimittel auf der Basis eines Estrogens und Progesteronantagonisten für die Hormonersatz-Therapie zu schaffen, welches eine stärkere antiproliferative Wirkung am Endometrium als die gemäß WO-A 93/17686 und WO-A 94/18983 beschriebenen Arzneimittel besitzt.

Diese Aufgabe wird durch die vorliegende Erfindung gelöst und zwar durch ein Arzneimittel enthaltend in Kombination einzelne Dosierungseinheiten eines Estrogens und einzelne Dosierungseinheiten eines kompetitiven Progesteronantagonisten zu dessen getrennter, sequentieller Verabreichung.
Dieses Arzneimittel kann für die Hormonersatz-Therapie verwendet werden.
Neben dem Arzneimittel betrifft die vorliegende Erfindung auch eine Packung, die dieses Arzneimittel enthält.

In dem erfindungsgemäßen Arzneimittel kommen die den Progesteronantagonisten enthaltenden Dosierungseinheiten also erst im Anschluß an eine bestimmte Zeitspanne, während der ausschließlich estrogenhaltige Dosierungseinheiten verabreicht wurden, zur Anwendung. Über den Zeitraum der Verabreichung des Progesteronantagonisten wird kein Estrogen gegeben. Durch diese sequentielle Gabe des Progesteronantagonisten wird eine ansonsten durch das Estrogen induzierte Proliferation des Endometriums gehemmt und außerdem das Ausmaß der Estrogen-abhängigen irregulären Blutungen verringert. Die Anwendung des Progesteronantagonisten führt also zu einem Schutz des Endometriums und induziert letztlich eine Amenorrhoe. Der schützende Effekt des Estrogens auf den Knochen bleibt dabei voll erhalten.

Es wurde gefunden, daß ein derartiges. Arzneimittel überraschenderweise eine stärkere antiproliferative Wirkung als die gemäß WO-A 93/17686 und WO-A 94/18983 hergestellten Arzneimittel aufweist.
Die stärkere antiproliferative Wirkung des erfindungsgemäßen Arzneimittels beruht auf der stärkeren antiproliferativen Wirkung des Progesteronantagonisten in Abwesenheit eines Estrogens als während einer gleichzeitigen Anwendung eines Progesteronantagonisten mit einem Estrogen (WO-A 94/18983). Das Risiko eines "unopposed estrogen effect" wird dadurch verringert.

Vorteile des erfindungsgemäßen Arzneimittels liegen darin, daß mit diesem die positiven Effekte des Estrogens auf den Knochen und die Lipide nicht gehemmt und keine Durchbruchblutungen induziert werden. Dies wird unter Vermeidung einer Mehrbelastung des Organismus, wie diese bei einer kombinierten Behandlung der Fall wäre, erreicht.

Es konnte gezeigt werden, daß bei ovarektomierten Cynomolgus-Affen (als Tiermodell für die postmenopausale Frau) die durch Estradiol stimulierte Proliferation des Myometriums bzw. Endometriums durch einen -nach ausschließlicher 28 tägiger Estrogengabe- über einen Zeitraum von 7 Tagen applizierten Progesteronantagonisten wie RU 486 inhibiert wird.

Die erfindungsgemäßen Arzneimittel sind sowohl für einen präventiven als auch für einen kurativen Einsatz in der Hormonersatz-Therapie (HRT: Hormone Replacement Therapy) geeignet, da durch das Estrogen ein Abbau von Knochenmasse verhindert wird, gleichzeitig das Estrogen protektiv auf das kardiovaskuläre System wirkt und die unerwünschten stimulierenden Effekte auf das Endometrium durch die antiproliferative Wirkung des Progesteronantagonisten verhindert werden.
Diese Arzmeimittet sind somit für eine Langzeitanwendung in der HRT besonders gut geeignet.

In dem erfindungsgemäßen Arzneimittel sind die Dosierungseinheiten des Estrogens zur Verabreichung vorzugsweise über einen Zeitraum von 28 bis 112 Tagen vorgesehen.

In einer weiteren Ausführungsform des erfindungsgemäßen Arzneimittels sind die Dosierungseinheiten des kompetitiven Progesteronantagonisten zur Verabreichung über einen Zeitraum von mindestens 4 Tagen und maximal 30 Tagen vorgesehen.

Eine besondere Ausfuhrungsförm des erfindungsgemäßen Arzneimittels enthält die Dosierungseinheiten des kompetitiven Progesteronantagonisten zur Verabreichung über einen Zeitraum von 7 Tagen.

Das erfindungsgemäße Arzneimittel ist vorzugsweise so ausgebildet, daß die Dosierungseinheiten des Estrogens und die Dosierungseinheiten des kompetitiven Progesteronantagonisten gemeinsam in einer solchen Anzahl in dem Arzneimittel vorliegen, daß die Summe aus der Anzahl der täglichen Dosierungseinheiten des Estrogens und der Dosierungseinheiten des kompetitiven Progesteronantagonistenes 28 oder 28 plus 7 oder 28 plus ein Mehrfaches von 7 beträgt.

Die Einnahme dieser Ausführungsform des erfindungsgemäßen Arzneimittels führt also zu einem exakt mehrwöchigen, mindestens aber 4wöchigen Verabreichungsryklus.
Als Beispiele können die folgenden Zusammensetzungen dienen:
28 Tageseinheiten Estrogen + 7 Tageseinheiten Progesteronantagonist, 28 Tageseinheiten Estrogen + 14 Tageseinheiten Progesteronantagonist, 28 Tageseinheiten Estrogen + 21 Tageseinheiten Progesteronantagonist, 56 Tageseinheiten Estrogen + 21 Tageseinheiten Progesteronantagonist etc.
Es sind aber genausogut bevorzugte Zusammensetzungen des erfindungsgemäßen Arzneimittels möglich, in denen die Anzahl der täglichen Dosierungseinheiten des Estrogens und die Anzahl der Dosierungseinheiten des kompetitiven Progesteronantagonistenen nicht jeweils ein Ein- oder Mehrfaches von 7 betragen: entscheidend ist nur, daß die Summe dieser Tageseinheiten durch 7 teilbar ist, d.h. die Einnahme des Armeimeittels zu einem exakt 4 oder mehrwöchigen Verabreichungszyklus führt.

Gemäß einer weiteren Ausführungsform liegt das Estrogen in zur täglichen, oralen Verabreichung bestimmten Dosierungseinheiten vor.

Ebenso kann der Progesteronantagonist in täglichen, oralen Dosierungseinheiten vorliegen.

Sind die Dosierungseinheiten des kompetitiven Progesteronantagonisten zur Verabreichung über einen Zeitraum von 7 Tagen vorgesehen, so können diese Dosienuigseinheiten vorteilhafterweise in Form einer wöchentlich einmalig zu verabreichenden Dosierungseinheit vorliegen.
In einer solchen wöchentlich einmalig zu verabreichenden Dosierungseinheit sollte der Progesteronantagonist vorzugsweise in einer Formulierung aufbereitet sein, die zu einer verzögerten Freisetzung des Wirkstoffes führt.
Eine verzögerte Freisetzung des kompetitiven Progesteronantagonisten läßt sich beispielsweise durch Formulierung der oral zu verabreichenden Dosierungseinheit als Matrixtablette oder durch Versehen der oral zu verabreichenden Dosierungseinheit mit einem retardierenden Überzug bewerkstelligen, wie dies dem Fachmann ohne weiteres bekannt ist
Es kann auch der zur Herstellung des erfindungsgemäßen Arzneimittels verwendete kompetitive Progesteronantagonist durch Derivatisierung, beispielsweise durch Veresterung einer freien Hydroxygruppe in einem wirksamen Vorläufer, eine längere Halbwertszeit als diese Vorstufe aufweisen. Dadurch wird ebenfalls eine länger anhaltende Wirkung erreicht. Dieses Prinzip ist beispielsweise in den in der EP-A 0 186 834 beschriebenen Estern des 11β-[4-N,N-(Dimethylamino)phenyl]-7α-hydroxy-17β-(3-hydroxypropyl)-13α-methyl-4,9(10)-gonadien-3-ons (Onapriston) verwirklicht.

Der estrogene Aspekt der vorliegenden Erfindung ist analog zur herkömmlichen Estrogenersatz-Therapie. Dementsprechend kann jede als Estrogen wirksame Verbindung in den bekannten Dosismengen und nach den für die Estrogenersatz-Therapie bekannten Methoden angewendet werden.

Als Estrogene kommen für die Zwecke der vorliegenden Erfindung alle estrogen wirksamen Verbindungen infrage.
Estrogene, die im Rahmen vorliegender Erfindung verwendet werden können, sind beispielsweise Ethinylestradiol, 17β-Estradiol sowie dessen Ester wie Estradiol-3-benzoat, Estradiol-17-valerat, -cypionat, -undecylat, -enanthat und/oder andere Estradiolester (US-PS 2,611,773, US-PS 2,990,414, US-PS 2,054,271, US-PS 2,225,419 und US-PS 2,156,599) und konjugierte Estrogene.
Auch Estradiol-, Ethinylestradiol- und Estron-3-sutfamate, beispielsweise Estron-N,N-dimethylsulfamat, Estron-N,N-diethylsulfamat, Ethinylesiradiol-3-N,N-dimethylsulfamat, Ethinylestradiol-3-N,N-diethylsulfamat, Ethinylestradiol-3-N,N-tetramethylensulfamat, Estronsulfamat, Estradiol-3-sulfamat, Estradiol-3-N,N-dimethylsulfamat, Estradiol-3-N,N-diethylsulfamat, Ethinylestradiol-3-sulfamat, die alle Prodrugs für die entsprechenden 3-Hydroxyverbindungen darstellen (W. Elger et al. in J. Steroid Biochem. Molec. Biol., Vol. 55, No. 3/4, 395-403, 1995; DE 44 29 398 Al und DE 44 29 397 Al), können in dem erfindungsgemäßen Arzneimittel verwendet werden.
Schließlich kommen auch noch die oral bioverfügbaren Derivate des 17β- und 17α-Estradiols mit einem modifizierten D-Ring des Steroidgerüst infrage.

Die Verwendung eines natürliches Estrogens (auch konjugierte Estrogene) oder ein Prodrug eines natürlichen Estrogens ist erfindungsgemäß bevorzugt.

Der Progesteronantagonist wird für die vorliegende Erfindung vorzugsweise ausgewählt aus der Gruppe der Verbindungen
11 1β-[4-(Dimethylamino)phenyl]-17β-hydroxy-17α-(1-propinyl)estra-4,9-dien-3-on (RU 38 486)
11β-[4-(Dimethylamino)phenyl]-17β-hydroxy-17α-(1-propinyl)-18a-homoestra-4,9-dien-3-on
11 β-[4-(Dimethylamino)phenyl]-17aβ-hydroxy-17aα-(1-propinyl)-17a-homoestra-4,9,16-trien-3-on
17α-Ethinyl-17β-hydroxy-11β-(4-methoxyphenyl)estra-4,9-dien-3-on
11β-(4-Acetylphenyl)-17β-hydroxy-17α-(1-propinyl)estra-4,9-dien-3-on
die 19,11β-überbrückten Steroide aus der EP-A 0 283 428,
die 10β-H-Steroide aus der EP-A 0 404 283, insbesondere
(Z)-11β-[4-(Dimethylamino)phenyl]-17β-hydroxy-17α-(3-hydroxy-1-propenyl)estr-4-en-3-on
11β-[4-(Dimethylamino)phenyl]-17α-hydroxy-17β-(3-hydroxypropyl)-13α-estra-4,9-dien-3-on (Onapriston)
(Z)-11β-(4-Acetylphenyl)-17β-hydroxy-17α-β-hydroxy-1-propenyl)estra-4,9-dien-3-on
(Z)-6'-(4-Cyanphenyl)-9,11α-dihydro-17β-hydroxy-17α-(3-hydroxy-1-propenyl)-4'*H-*naphth[3',2',1':10,9,11]estra-4,9(11)-dien-3-on
(Z)-9,11α-Dihydro-17β-hydroxy-17α-(3-hydroxy-1-propenyl)-6'-(3-pyridinyl)-4'*H-*naphth[3',2',1':10,9,11]estra-4,9(11)-dien-3-on
4',5'-Dihydro-11β-[4-(dimethylamino)phenyl]-6β-methylspiro[estra-4,9-dien-17β,2'(3'*H*)-furan]-3-on
4',5'-Dihydro-11β-[4-(dimethylamino)phenyl]-7β-methylspiro[estra-4,9-dien-17β,2'(3'*H*)-furan]-3-on
11β-(4-Acetylphenyl)-19,24-dinor-17,23-epoxy-17α-chola-4,9,20-trien-3-on.

Insbesondere bevorzugt sind für die Zwecke vorliegender Erfindung die Progesteronantagonisten
11β-[4-(Dimethylamino)phenyl]-17β-hydroxy-17α-(1-propinyl)estra-4,9-dien-3-on (RU 38 486)
(Z)-11β-[4-(Dimethytamino)phenyl]-17β-hydroxy-17α-(3-hydroxy-1-propenyl)estr-4-en-3-on
4',5'-Dihydro-11β-[4-(dimethylamino)phenyl]-6β-methylspiro[estra-4,9-dien-17β,2'(3*H*)-furan]-3-on
4',5'-Dihydro-11**β**-[4-(dimethylamino)phenyl]-7**β**-methylspiro[estra-4,9-dien-17β,2'(3'*H*)-furan]-3-on
11β-(4-Acetylphenyl)-19,24-dinor-17,23-epoxy-17**α**-chola-4,9,20-trien-3-on.

Die Formulierung des Estrogens und des Progesteronantagonisten erfolgt für die Zwecke der vorliegenden Erfindung vollkommen konventionell, wie es bereits für die Formulierung dieser Verbindungen für deren Einzeianwendung in der Hormonersatz-Therapie für Estrogen, bspw. Cycloprogynova, bzw. in der Tumortherapie oder für den Schwangerschaftsabbruch für Progesteronantagonisten, bspw. Mifepristone, bekannt ist.
Insbesondere wird auch auf die in den WO-A 93/17686 und WO-A 94/18983 enthaltenen Angaben verwiesen.

Neben der oralen Verabreichung des Estrogens und des Progesteronantagonisten, ist es genausogut möglich, eine oder beide der Komponenten transdermal, bspw. mit einem Hautpflaster, zu verabreichen, die für die Applikation von Estrogenen bestens bekannt sind (Climara-Patch).

Des weiteren ist die Verabreichung mittels eines intrauterinen Freisetzungssystems möglich, doch ist diese Variante im Rahmen vorliegender Erfindung nicht bevorzugt.

Es ist auch die Verabreichung einer der Komponenten oder auch beider als Depotformuiierung möglich.

Schließlich können alle genannten Applikationsarten kombiniert werden. Beispielsweise kann das Estrogen transdermal mit einem Hautpflaster appliziert und der Progesteronantagonist täglich oral oder ein- oder mehrmalig als Depotformulierung verabreicht werden.

Das Estrogen ist pro täglicher Dosierungseinheit erfindungsgemäß in einer Menge von 1 bis 2 mg Estradiol oder einer bioequivalenten Menge eines anderen Estrogens enthalten.
Als bioequivalente Mengen anderer Estrogene für die Zwecke vorliegender Erfindung sind folgende Mengen anzusehen:
Ethinylestradiol 5 - 35 µg
konjugierte Estrogene 0,625 bis 1,25 mg.
Im Falle einer transdermalen Applikation des Estrogens sollte das transdermale Applikationssystem täglich ungefähr 50 µg Estradiol oder eine bioequivalente Menge eines anderen Estrogens freisetzen.
Auch die Applikation des Estrogens mittels einer Vagmalcreme oder Vaginalringes ist möglich. Die täglichen Mengen liegen im Fall von Estradiol um 1,25 mg bzw. 0,2 mg. Hierbei handelt es sich um Orientierungswerte.

Der kompetitive Progesteronantagonist ist in dem erfindungsgemäßen Arzneimittel in jeder Dosierungseinheit vorzugsweise in einer solchen Menge enthalten, die bei Anwendung über die vorgesehene Zeitspanne zur Ausbildung einer Amenorrhoe ausreichend ist.

In einer besonders bevorzugten Ausführungsform des erfindungsgemäßen Arzneimittels ist der kompetitive Progesteronantagonist je täglicher Dosierungseinheit in einer Menge enthalten, die 0,5 mg bis 10 mg bevorzugt 1mg bis 5 mg RU 486 äquivalent ist.

Die das erfindungsgemäße Arzneimittel enthaltende Packung ist so hergerichtet, daß sie außer den beiden Komponenten Estrogen und Progesteronantagonist in der jeweils beabsichtigten Applikationsform (oral in Form von Pillen, Dragees etc. in einem Blister, was für das Estrogen und/oder den Progesteronantagonisten zutreffen kann, oder das Estrogen als Haupflaster und der Progesteronantagonist in Form von Pillen, Dragees etc. in einem Blister oder in einer Kapsel als ein einmalig zu verabreichendes Depot) noch Hinweise für die Anwendung des Arzneimittels (Beipackzettel) enthält.

## Patentansprüche

1. Arzneimittel enthaltend in Kombination einzelne Dosierungseinheiten eines Estrogens und einzelne Dosierungseinheiten eines kompetitiven Progesteronantagonisten zu dessen getrennter, sequentieller Verabreichung, wobei die Verabreichung der einzelnen Dosierungseinheiten des Estrogens zuerst erfolgt und von der Verabreichung der einzelnen Dosienmgseinheiten des kompetitiven Progesteronantagonisten gefolgt wird, und die in Abwesenheit des Estrogens erfolgt.

2. Arzneimittel nach Anspruch 1, worin die Dosierungseinheiten des Estrogens zur Verabreichung über einen Zeitraum yon 28 bis 90 Tagen vorgesehen sind.

3. Arzneimittel nach Anspruch 1, worin die Dosierungseinheiten des Estrogens zur Verabreichung über einen Zeitraum von 28 bis 30 Tagen vorgesehen sind.

4. Arzneimittel nach Anspruch 1, worin die Dosierungseinheiten des Estrogens zur oralen Verabreichung hergerichtet sind.

5. Arzneimittel nach Anspruch 1, worin die Dosierungseinheiten des Estrogens zur transdermalen Applikation hergerichtet sind.

6. Arzneimittel nach Anspruch 1, worin die Dosierungseinheiten des kompetitiven Progesteronantagonisten zur Verabreichung über einen Zeitraum von mindestens 4 Tagen bis maximal 30 Tagen vorgesehen sind.

7. Arzneimittel nach Anspruch 6, worin die Dosierungseinheiten des kompetitiven Progesteronantagonisten zur Verabreichung über einen Zeitraum von 7 Tagen vorgesehen sind.

8. Arzneimittel nach Anspruch 7, worin die Dosierungseinheiten des Estrogens zur Verabreichung über einen Zeitraum von 28 bis 30 Tagen vorgesehen sind.

9. Arzneimitteln ach Anspruch 7, worin die Dosierungseinheiten des Estogens zur Verabreichung über einen Zeitraum von 60 Tagen vorgesehen sind.

10. Arzneimittel nach Anspruch 5, worin die Dosierungseinheiten des kompetitiven Progesteronantagonisten zur Verabreichung über einen Zeitraum von 20 Tagen vorgesehen sind.

11. Arzneimittel nach einem der vorstehenden Ansprüche, **dadurch gekennzeichnet, daß** die Dosierungseinheiten des Estrogens und die Dosierungseinheiten des kompetitiven Progesteronantagonisten gemeinsam in einer solchen Anzahl in dem Arzneimittel vorliegen, daß die Summe aus der Anzahl der täglichen Dosierungseinheiten des Estrogens und der Dosierungseinheiten des kompetitiven Progesteronantagonistenes 28 oder 28 plus 7 oder 28 plus ein Mehrfaches von 7 beträgt.

12. Arzneimittel nach Anspruch 1, worin die Dosierungseinheiten des Progesteronantagonisten zur oralen Verabreichung hergerichtet sind.

13. Arzneimittel nach Anspruch 1, worin die Dosierungseinheiten des Progesteronantagonisten als ein- oder mehrmalig zu verabreichende Depotformulierung vorliegen.

14. Arzneimittel nach Anspruch 1, worin jede einzelne Dosierungseinheit des Estrogens eine tägliche Dosierungseinheit ist.

15. Arzneimittel nach Anspruch 1, worin jede einzelne Dosierungseinheit des kompetitiven Progesteronantagonisten eine tägliche Dosierungseinheit ist.

16. Arzneimittel nach Anspruch 7, worin diese Dosierungseinheiten in Form einer wöchentlich einmalig zu verabreichenden Dosierungseinheit vorliegen.

17. Arzneimittel nach Anspruch 1, worin das Estrogen ausgewählt ist aus der Gruppe der Verbindungen
Ethinylestradiol, 17β-Estradiol, Estradiol-3-benzoat, Estradiol-17-valerat, -cypionat, - undecylat, -enanthat, konjugierte Estrogene, Estron-N,N-dimethylsulfamat, Estron-N,N-diethylsulfamat, Ethinylestradiol-3-N,N-dimethylsulfamat, Ethinylestradiol-3-N,N-diethylsulfamat, Ethinylestradiol-3-N,N-tetramethylensulfamat, Estronsulfamat, Estradiol-3-sulfamat, Estradiol-3-N,N-dimethylsulfamat, Estradiol-3-N,N-diethylsulfamat, Ethinylestradiol-3-sulfamat.

18. Arzneimittel nach Anspruch 17, worin das Estrogen ein natürliches Estrogen ist.

19. Arzneimittel nach Anspruch 1, worin der kompetitive Progesteronantagonist ausgewählt ist aus der Gruppe der Verbindungen
11β-[4-(Dimethylamino)phenyl]-17β-hydroxy-17α-(1-propinyl)estra-4,9-dien-3-on (RU 486)
11β-[4-(Dimethylamino)phenyl]-17β-hydroxy-17α-(1-propinyl)-18a-homoestra-4,9-dien-3-on
11β-[4-(Dimethylamino)phenyl]-17aβ-hydroxy-17aα-(1-propinyl)-17a-homoestra-4,9,16-trien-3-on
17α-Ethinyl-17β-hydroxy-11β-(4-methoxyphenyl)estra-4,9-dien-3-on
11β-(4-Acetylphenyl)-17β-hydroxy-17α-(1-propinyl)estra-4,9-dien-3-on
11β-[4-(Dimethylamino)phenyl]-17α-hydroxy-17β(3-hydroxypropyl)-13α-estra-4,9-dien-3-on (Onapriston)
(Z')-11β-[4-(Dimethylamino)phenyl]-17β-hydroxy-17α-(3-hydroxy-1-propenyl)estra-4-en-3-on
(Z)-11β-(4-Acetylphenyl)-17β-hydroxy-17α-(3-hydroxy-1-propenyl)estra-4,9-dien-3-on
(Z)-6'-(4-Cyanphenyl)-9,11α-dihydro-17β-hydroxy-17α-(3-hydroxy-1-propenyl)-4'*H-*naphth[3',2',1':10,9,11]estra-4,9(11)-dien-3-on
(Z)-9,11α-Dihydro-17β-hydroxy-17α-(3-hydroxy-1-propenyl)-6'-(3-pyridinyl)-4'*H-*naphth[3',2',1':10,9,11]estra-4,9(11)-dien-3-on
4',5'-Dihydro-11β-[4-(dimethylamino)phenyl]-6β-methylspiro[estra-4,9-dien-17β,2'(3'*H*)-furan]-3-on
4',5'-Dihydro-11β-[4-(dimethylamino)phenyl]-7β-methylspiro[estra-4,9-dien-17β,2'(3'*H*)-furan]-3-on
11β-(4-Acetylphenyl)-19,24-dinor-17,23-epoxy-17α-chola-4,9,20-trien-3-on.

20. Arzneimittel nach Anspruch 19, worin der kompetitive Progesteronantagonist
11β-[4-(Dimethylamino)phenyl]-17β-hydroxy-17α-(1-propinyl)estra-4,9-dien-3-on (RU 38 486)
(Z)-11β-[4-(Dimethylamino)phenyl]-17β-hydroxy-17α-(3-hydroxy-1-propenyl)estr-4-en-3-on
4',5'-Dihydro-11β-[4-(dimethylamino)phenyl]-6β-methylspiro[estra-4,9-dien-17β,2'(3'*H*)-furan]-3-on
4',5'-Dihydro-11β-[4-(dimethylamino)phenyl]-7β-methylspiro[estra-4,9-dien-17β,2'(3'*H*)-furan]-3-on
11β-(4-Acetylphenyl)-19,24-dinor-17,23-epoxy-17α-chola-4,9,20-trien-3-on ist.

21. Arzneimittel nach Anspruch 1, worin das Estrogen in einer Menge von 1 bis 2 mg Estradiol oder einer bioequivalenten Menge eines anderen Estrogens je täglicher Dosierungseinheit enthalten ist.

22. Arzneimittel nach Anspruch 1, worin der kompetitive Progesteronantagonist in einer eine Amenorrhoe auslösenden Menge enthalten ist.

23. Arzneimittel nach Anspruch 22, worin der kompetitive Progesteronantagonist je täglicher Dosierungseinheit in einer Menge enthalten ist, die 0,5 mg bis 10 mg RU 486 äquivalent ist.

24. Arzneimittel nach einem der vorstehenden Ansprüche 1 bis 23 zur Verwendung für die Hormonersatz-Therapie.

25. Packung für die Hormonersatz-Therapie enthaltend einzelne Dosierungseinheiten eines Estrogens und einzelne Dosierungseinheiten eines kompetitiven Progesteronantagonisten zu dessen getrennter, sequentieller Verabreichung gemäß einem der vorstehenden Ansprüche 1 bis 24 sowie Angaben zur Applikation der Dosierungseinheiten des Estrogens oder des Antiestrogens mit partial-agonistischer Wirkung und der Dosierungseinheiten des kompetitiven Progesteronantagonisten.

## Claims

1. Medicament comprising in combination individual dosage units of an estrogen and individual dosage units of a competitive progesterone antagonist for separate, sequential administration thereof, where administration of the individual dosage units of the estrogen takes place first and is followed by administration of the individual dosage units of the competitive progesterone antagonist, and which takes place in the absence of the estrogen.

2. Medicament according to Claim 1, in which the estrogen dosage units are provided for administration over a period of 28 to 90 days.

3. Medicament according to Claim 1, in which the estrogen dosage units are provided for administration over a period of 28 to 30 days.

4. Medicament according to Claim 1, in which the estrogen dosage units are designed for oral administration.

5. Medicament according to Claim 1, in which the estrogen dosage units are designed for transdermal administration.

6. Medicament according to Claim 1, in which the dosage units of the competitive progesterone antagonist are provided for administration over a period of at least 4 days to not more than 30 days.

7. Medicament according to Claim 6, in which the dosage units of the competitive progesterone antagonist are provided for administration over a period of 7 days.

8. Medicament according to Claim 7, in which the estrogen dosage units are provided for administration over a period of 28 to 30 days.

9. Medicament according to Claim 7, in which the estrogen dosage units are provided for administration over a period of 60 days.

10. Medicament according to Claim 5, in which the dosage units of the competitive progesterone antagonist are provided for administration over a period of 20 days.

11. Medicament according to any of the preceding claims, **characterized in that** the estrogen dosage units and the dosage units of the competitive progesterone antagonist are present together in the medicament in a number such that the total of the number of daily estrogen dosage units and of the dosage units of the competitive progesterone antagonist is 28 or 28 plus 7 or 28 plus a multiple of 7.

12. Medicament according to Claim 1, in which the dosage units of the progesterone antagonist are designed for oral administration.

13. Medicament according to Claim 1, in which the dosage units of the progesterone antagonist are present as depot formulation to be administered one or more times.

14. Medicament according to Claim 1, in which each individual estrogen dosage unit is a daily dosage unit.

15. Medicament according to Claim 1, in which each individual dosage unit of the competitive progesterone antagonist is a daily dosage unit.

16. Medicament according to Claim 7, in which these dosage units are present in the form of a dosage unit to be administered once a week,

17. Medicament according to Claim 1, in with the estrogen is selected from the group of compounds ethinylestradiol, 17β-estradiol, estradiol 3-benzoate, estradiol 17-valerate, -cypionate, -undecylate, -oenanthate, conjugated estrogens, estrone N,N-dimethylsulphamate, estrone N,N-diethylsulphamate, ethinylestradiol 3-N,N-dimethylsulphamate, ethinylestradiol 3-N,N-diethylsulphamate, ethinylestradiol 3-N,N-tetramethylenesulphamate, estrone sulphamate, estradiol 3-sulphamate, estradiol 3-N,N-dimethylsulphamate, estradiol 3-N,N-diethylsulphamate, ethinylestradiol 3-sulphamate.

18. Medicament according to Claim 17, in which the estrogen is a natural estrogen.

19. Medicament according to Claim 1, in which the competitive progesterone antagonist is selected from the group of compounds
11β- [4-(dimethylamino) phenyl]-17β-hydroxy-17α- (1-propynyl)estra-4,9-dien-3-one (RU 486)
11β-[4-(dimethylainino)phenyl]-17β-hydroxy-17α-(1-propynyl)-18a-homoestra-4,9-dien-3-one
11β- [4- (dimethylamino) phenyl] -17aβ-hydroxy-17aα-(1-propynyl)-17a-homoestra-4,9,16-trien-3-one
17α-ethynyl-17β-hydroxy-11β-(4-methoxyphenyl)estra-4,9-dien-3-one
11β-(4-acetylphenyl)-17β-hydroxy-17α-(1-propynyl)estra-4, 9-dien-3-one
11β-[4-(dimethylamino)phenyl]-17α-hydroxy-17β-(3-hydroxypropyl)-13α-estra-4,9-dien-3-one (onapristone)
(Z)-11β-[4-(dimethylamino) phenyl]-17β-hydroxy-17α-(3-hydroxy-1-propenyl)estr-4-en-3-one
(2)-11β-(4-acetylphenyl)-17β-hydroxy-17α-(3-hydroxy-1-propenyl)estra-4,9-dien-3-one
(Z)-6'-(4-cyanophenyl)-9,11α-dihydro-17β-hydroxy-17α-(3-hydroxy-1-propenyl)-4'H-naphth[3',2',1':10,9,11]-estra-4,9(11)-dien-3-one
(Z)-9,11α-dihydro-17β-hydroxy-17α-(3-hydroxy-1-propenyl)-6'-(3-pyridinyl)-4'H-naphth[3',2',1':10,9,11]estra-4,9(11)-dien-3-one
4'5'-dihydro-11β-[4-(dimethylamino)phenyl]-6β-methylspiro[estra-4,9-diene-17β,2'(3'H)-furan]-3-one
4',5'-dihydro-11β-[4-(dimethylamino)phenyl]-7β-methylspiro[estra-4,9-diene-17β,2'(3'H)-furan]-3-one
11β-(4-acetylphenyl)-19,24-dinor-17,23-epoxy-17α-chola-4,9,20-trien-3-one.

20. Medicament according to Claim 19, in which the competitive progesterone antagonist is
11β-[4-(dimethylamino)phenyl] -17β-hydroxy-17α-(1-propynyl)estra-4,9-dien-3-one (RU 38 486)
(Z)-11β-[4-(dimethylamino)phenyl]-17β-hydroxy-17α-(3-hydroxy-1-propenyl)estr-4-en-3-one
4',5'-dihydro-11β-[4-(dimethylamino)phenyl]-6β-methylspiro[estra-4,9-diene-17β,2'(3'H)-furan]-3-one
4',5'-dihydro-11β-[4-dimethylamino)phenyl]-7β-methylspiro[estra-4,9-diene-17β,2'(3'H)-furan]-3-one
11β-(4-acetylphenyl)-19,24-dinor-17,23-epoxy-17α-chola-4,9,20-trien-3-one.

21. Medicament according to Claim 1, in which the estrogen is present in an amount of from 1 to 2 mg of estradiol or a bioequivalent amount of another estrogen in each daily dosage unit.

22. Medicament according to Claim 1, in which the competitive progesterone antagonist is present in an amount which induces amenorrhoea.

23. Medicament according to Claim 22, in which the competitive progesterone antagonist is present in an amount which is equivalent to from 0.5 mg to 10 mg of RU 486 in each daily dosage unit.

24. Medicament according to any of preceding Claims 1 to 23 for use for hormone replacement therapy.

25. Pack for hormone replacement therapy comprising individual dosage units of an estrogen and individual dosage units of a competitive progesterone antagonist for separate, sequential administration thereof according to any of preceding Claims 1 to 24, and information on administration of dosage units of the estrogen or of the antiestrogen having a partial agonistic effect and of the dosage units of the competitive progesterone antagonist.

## Revendications

1. Médicament contenant en association des doses unitaires individuelles d'un oestrogène et des doses unitaires individuelles d'un antagoniste compétitif de progestérone pour leur administration séparée, séquentielle, l'administration des doses unitaires individuelles de l'oestrogène s'effectuant d'abord et étant suivie de l'administration des doses unitaires individuelles de l'antagoniste compétitif de progestérone, et qui s'effectue en absence de l'oestrogène.

2. Médicament selon la revendication 1, dans lequel les doses unitaires de l'oestroqène sont prévues pour l'administration sur une période de 28 à 90 jours.

3. Médicament selon la revendication 1, dans lequel les doses unitaires de l' oestrogène sont prévues pour l'administration sur une période de 28 à 30 jours.

4. Médicament selon la revendication 1, dans lequel les doses unitaires de l'oestrogène sont destinées à l'administration par voie orale.

5. Médicament selon la revendication 1, dans lequel les doses unitaires de l'oestrogène sont destinées à l'administration transdermique.

6. Médicament selon la revendication 1, dans lequel les doses unitaires de l'antagoniste compétitif de progestérone sont prévues pour l'administration sur une période d'au moins 4 jours à 30 jours au maximum.

7. Médicament selon la revendication 6, dans lequel les doses unitaires de l'antagoniste compétitif de progestérone sont prévues pour l'administration sur une période de 7 jours.

8. Médicament selon la revendication 7, dans lequel les doses unitaires de l'oestrogène sont prévues pour l'administration sur une période de 28 à 30 jours.

9. Médicament selon la revendication 7, dans lequel les doses unitaires de l' oestrogène sont prévues pour l'administration sur une période de 60 jours.

10. Médicament selon la revendication 5, dans lequel les doses unitaires de l'antagoniste compétitif de progestérone sont prévues pour l'administration sur une période de 20 jours.

11. Médicament selon l'une quelconque des revendications précédentes, **caractérisé en ce que** les doses unitaires de l'oestrogène et les doses unitaires de l'antagoniste compétitif de progestérone sont présentes conjointement dans le médicament en un nombre tel que la somme du nombre des doses unitaires journalières de l'oestrogène et des doses unitaires de l'antagoniste compétitif de progestérone est égale à 28 ou 28 plus 7 ou 28 plus un multiple de 7.

12. Médicament selon la revendication 1, dans lequel les doses unitaires de l'antagoniste de progestérone sont destinées à l'administration par voie orale.

13. Médicament selon la revendication 1, dans lequel les doses unitaires de l'antagoniste de progestérone se trouvent sous forme d'une composition retard à administrer une ou plusieurs fois.

14. Médicament selon la revendication 1, dans lequel chaque dose unitaire individuelle de l'oestrogène est une dose unitaire journalière.

15. Médicament selon la revendication 1, dans lequel chaque dose unitaire individuelle de l'antagoniste compétitif de progestérone est une dose unitaire journalière.

16. Médicament selon la revendication 7, dans lequel ces doses unitaires se trouvent sous forme d'une dose unitaire à administrer une fois par semaine,

17. Médicament selon la revendication 1, dans lequel l'oestrogène est choisi dans le groupe de composés éthynylestradiol, 17β-estradiol, 3-benzoate d'estradiol, 17-valérate, -cypionate, -undécylate, -énanthate d'estradiol, estrogènes conjugués, N,N-diméthylsulfamate d'estrone, N,N-diéthylsulfamate d'estrone, 3-N,N-diméthylsulfamate d'éthynylestradiol, 3-N,N-diéthylsulfamate d'éthynylestradiol, 3-N,N-tétraméthylènesulfamate d'éthynylestradiol, sulfamate d'estrone, 3-sulfamate d'estradiol, 3-N,N-diméthylsulfamate d'estradiol, 3-N,N-diéthylsulfamate d'estradiol, 3-sulfamate d'éthynylestradiol.

18. Médicament selon la revendication 17, dans lequel l'oestrogène est un oestrogène naturel.

19. Médicament selon la revendication 1, dans lequel l'antagoniste compétitif de progestérone est choisi dans le groupe des composés
11β-[4-(diméthylamino)phényl]-17β-hydroxy-17α-(1-propynyl)estra-4,9-dién-3-one (RU 486)
11β-[4-(diméthylamino)phényl]-17β-hydroxy-17α-(1-propynyl)-18a-homoestra-4,9-dién-3-one
11β-[4-(diméthylamino)phényl]-17aβ-hydroxy-17aα-(1-propynyl)-17a-homoestra-4,9,16-trién-3-one
17α-éthynyl-17β-hydroxy-11β-(4-méthoxyphényl)-estra-4,9-dién-3-one
11β-(4-acétylphényl)-17β-hydroxy-17α-(1-propynyl)-estra-4,9-dién-3-one
11β-[4-(diméthylamino)phényl]-17α-hydroxy-17β-(3-hydroxypropyl)-13α-estra-4,9-dién-3-one (onapristone)
(Z)-11β-[4-(diméthylamino)phényl]-17β-hydroxy-17α-(3-hydroxy-1-propényl)estra-4-én-3-one
(Z)-11β-(4-acétylphényl)-17β-hydroxy-17α-(3-hydroxy-1-propényl)estra-4,9-dién-3-one
(Z)-6'-(4-cyanophényl)-9,11α-dihydro-17β-hydroxy-17α-(3-hydroxy-1-propényl)-4'H-napht-[3',2',1':10,9,11]estra-4.9(11)-dién-3-one
(Z)-9,11a-dxhydro-17α-hydroxy-17α-(3-hydroxy-1-propényl)-6'-(3-pyridinyl)-4'H-napht-[3',2',1':10,9,11]estra-4,9(11)-dién-3-one
4',5'-dihydro-11β-[4-(diméthylamino)phényl]-6β-méthylspiro[estra-4,9-diéne-17β,2'(3'H)-furann]-3-one
4',5'-dihydro-11β-[4-(diméthylamino)phényl-7β-méthylspiro[estra-4,9-dièn-17β,2'(3'H)-furann]-3-one
11β-(4-acétylphényl)-19,24-dinor-17,23-époxy-17α-chola-4,9,20-trién-3-one.

20. Médicament selon la revendication 19, dans lequel l'antagoniste compétitif de progestérone est
la 11β-[4-(diméthylamino)phényl]-17β-hydroxy-17α-(1-propynyl)estra-4,9-dién-3-one (RU 38 486)
la (Z)-11β-[4-(diméthylamino)phényl]-17β-hydroxy-17α-(3-hydroxy-1-propényl)estra-4-én-3-one
la 4',5'-dihydro-11β-[4-(diméthylamino)phényl-6β-méthylspiro[estra-4,9-dièn-17β,2'(3'H)-furann]-3-one
la 4',5'-dihydro-11β-[4-(diméthylamino)phényl]-7β-méthylspiro[estra-4,9-dièn-17β,2' (3'H)-furann]-3-one
la 11β-(4-acétylphényl)-19,24-dinor-17,23-époxy-17α-chola-4,9,20-trién-3-one.

21. Médicament selon la revendication 1, dans lequel l'oestrogène est contenu en une quantité de 1 à 2 mg d'estradiol ou en une quantité biologiquement équivalente d'un autre oestrogène, par dose unitaire journalière.

22. Médicament selon la revendication 1, dans lequel l'antagoniste compétitif de progestérone est contenu en une quantité déclenchant une aménorrhée.

23. Médicament selon la revendication 22, dans lequel l'antagoniste compétitif de progestérone est contenu, par dose unitaire journalière, en une quantité qui est équivalente à 0,5 mg à 10 mg de RU 486.

24. Médicament selon l'une quelconque des revendications précédentes 1 à 23, pour l'utilisation pour l'hormonothérapie substitutive.

25. Conditionnement pour l'hormonothérapie substitutive, contenant des doses unitaires individuelles d'un oestrogène et des doses unitaires individuelles d'un antagoniste compétitif de progestérone pour leur administration séparée, séquentielle selon l'une quelconque des revendications 1 à 24 ainsi que des indications pour l'administration des doses unitaires de l'oestrogène ou de l'anti-oestrogène à action agoniste partielle et des doses unitaires de l'antagoniste compétitif de progestérone.
